# EUROPEAN PATENT APPLICATION

(11) **EP 2 623 537 A1**
(43) Date of publication of application: **07.08.2013**
(21) Application number: 12000672.1
(22) Date of filing: 01.02.2012
(51) Int. Cl.: C08G 63/02, C08G 63/78, C08G 63/87, C09J 167/00, C12P 7/62

(54) **Enzyme-catalyzed polycondensate method production and adhesive composition**

(71) Applicant: NITTO EUROPE N.V, 3600 Genk (BE); Nitto Denko Corporation, Osaka 567-8680 (JP)
(72) Inventor: Van Der Meulen, Inge, 5504 MJ Veldhoven (NL); Noordover, Bart Adrianus Johannes, 5643 SB Eindhoven (NL); Eevers, Walter, 3520 Zonhoven (BE); Shigematsu, Takayuki, 3500 Hasselt (BE); Vendamme, Richard, 3500 Hasselt (BE)
(74) Representative: Grünecker, Kinkeldey, Stockmair & Schwanhäusser

(57) **Abstract**

A polycondensate obtainable by reacting: (a) a carboxyl-terminated oligomeric condensation product of (a1) a polyol comprising at least three hydroxyl groups, wherein at least two of these hydroxyl groups are primary hydroxyl groups, and (a2) a dicarboxylic acid, and (b) a diol, comprising two primary hydroxyl groups, wherein the condensation reaction between (a) and (b) is catalyzed by an enzyme. Furthermore, the present invention provides a method of producing such a polycondensate and adhesive compositions comprising said polycondensate.

## Description

The present invention relates to an enzyme-catalyzed polycondensate, a method of its production and adhesive compositions comprising said polycondensate.

Typical polycondensates are polyesters, which are used in various adhesive applications, such as pressure-sensitive adhesive tapes (PSA) or hot-melt films. The polycondensation reaction to produce polyesters usually requires elevated temperatures. In particular, polyesters can be formed by a direct polycondensation of dicarboxylic acids and diols at temperatures of approximately 150°C to 250°C. The polycondensation of dicarboxylic acids and diols generally leads to polyesters showing a low functionality due to the fact that the functional groups, i.e. the hydroxyl and/or carboxyl groups, are exclusively located at the end of the polymer chains, whereas the polymer backbone does not contain functional groups. This low functionality is associated with disadvantages, such as low hydrophilicity or low biodegradability. Furthermore, such polyesters may not be adequately crosslinked due to the absence of these functional groups in the polymer backbone. One attempt to solve this drawback, i.e. the create functional groups in the backbone of the polycondensate, is the incorporation of polyfunctional monomers, such as polyols, in the polyester.

It is known to conduct the polycondensation reaction in the presence of esterification or transesterification catalysts, such as e.g. titanium tetrabutoxide, which facilitates a reaction at lower temperatures. However, the polycondensation of dicarboxylic acid with polyols is very difficult to control with such standard catalysts, since such a reaction usually generates highly branched polyesters due to the fact that these catalysts unselectively catalyze the esterification of all hydroxyl groups of the polyol.

In contrast to such standard catalysts, it is known that specific enzymes, such as lipases, can selectively catalyze the esterification of carboxylic acids with low sterically hindered hydroxyl groups, in particular primary hydroxyl groups, under mild conditions. Thus, such an enzyme-catalyzed polycondensation leads to the formation of predominantly linear polyesters from dicarboxylic acids and polyols. US 6,972,315 B2 shows various polycondensates derived from enzyme-catalyzed polycondensation reactions by a one-step reaction process. WO 94/12652 also teaches a method for producing polyesters with an enzymatic process.

However, the methods disclosed in these documents are improvable since they are not suitable for sufficiently incorporating any combination of monomers in the polyester. In particular, these known processes are not suitable for incorporating monomers, such as dicarboxylic acids, diol and polyols, with different polarities in the polycondensate. This, on the other hand, would be desirable for adjusting the polymer properties, particularly for using said polycondensates in adhesive compositions.

Thus, it is an object of the present invention to provide a polycondensate, which can incorporate all kinds of monomers as repeating units, i.e. especially monomers with different polarities. A further object is the provision of a polycondensate that comprises a sufficient amount of functional groups, such as hydroxyl groups, in the polymer backbone. This can facilitate a sufficient degree of subsequent crosslinking and ensures an enhanced hydrophilicity, water solubility and biodegradability. These objects are solved by the polycondensate as defined in claim 1. Preferred embodiments are specified in claims 2 to 11.

A further object is the provision of a method for the production of a polycondensate, showing these adventurous properties, under mild conditions, such as temperatures of 50°C or lower. This object is solved by the method for producing a polycondensate in accordance with claim 12. A preferred embodiment is defined in claim 13.

Additional objects are the provision of adhesive compositions, such as a pressure sensitive adhesive (PSA) composition or hot melt adhesive composition with tailor made properties, which are suitable for various applications, such as PSA tapes or hot-melt films. These objects are solved by the adhesive compositions in accordance with claims 14 and 15.

Preferably and advantageously the polycondensates/polyester compositions in accordance with the present invention are prepared without the use of metal-based catalysts. Metal-free polymers are required for various applications in the biomedical and medical fields, due to the potentially toxic nature of metal-based catalysts.

Another advantage of the polycondensates/polyester compositions in accordance with the present invention is the fact that they show enhanced stability since they do not contain residual metal-based catalyst (or residues of such metal-based catalysts). Despite the fact that metal-based catalysts are highly efficient, they should be used with caution due to the important degradation that they potentially induce to the polymer (yellowing, reduced viscosity upon aging). Such problems can be prevented with the enzymatic process.

Any combination of preferred embodiments defined in the sub claims or the specification are encompassed by the present invention. Further objects of the invention will be apparent from the description.

A polycondensate in accordance with the present invention is obtainable by reacting: (a) a carboxyl-terminated oligomeric condensation product of (a1) a polyol comprising at least three hydroxyl groups, wherein at least two of these hydroxyl groups are primary hydroxyl groups, and (a2) a dicarboxylic acid, and (b) a diol, comprising two primary hydroxyl groups, wherein the condensation reaction between (a) and (b) is catalyzed by an enzyme.

In other words, the polycondensate is obtainable by enzymatically condensating the carboxyl groups of (a) a carboxyl-terminated oligomeric condensation product and (b) a diol to form a polyester. Said carboxyl-terminated oligomeric condensation product is produced in a previous reaction step by condensating (a1) a polyol comprising at least three hydroxyl groups, wherein at least two of these hydroxyl groups are primary hydroxyl groups, and (a2) a dicarboxylic acid.

More specifically, the polycondensate is produced in by two-step method, comprising a first step of producing a carboxyl-terminated an oligomeric condensation product (a) from a polyol (a1) and a dicarboxylic acid (a2), and a second step of producing a polycondensate from (a) and (b). The inventive two-step method also has the advantage that a huge variety of monomers can be incorporated in the resulting polycondensate, independently from the nature of these monomers. Such a two-step method enables incorporating relatively hydrophobic monomers, such as dimers of fatty acid/alcohol, in combination with relatively hydrophilic monomers, such as the polyol or further hydrophilic diols / dicarboxylic acids, in the polycondensate.

In contrast thereto, a condensation reaction by a known one-step method, wherein all monomers are simultaneously present in the reaction mixture, does not result in a polycondensate in accordance with the present invention. This can be explained by the fact the monomers usually show a different polarity i.e. hydrophilicity / hydrophobicity, which results in a different compatibility, to each other. This, on the other hand, affects their incorporation in the polycondensate, which leads to differences in the composition of the polycondensate and of the resulting molecular weight. Furthermore, it is apparent that the microstructure of the polycondensate produced of by a two-step method in accordance with the present invention differs from a polycondensate that is produced by a known one-step method.

Moreover, it is crucial for the present invention that the condensation reaction between (a) and (b) is catalyzed by an enzyme. The enzyme may be any enzyme that is capable of catalyzing the condensation reaction between a carboxyl group and a hydroxyl group. In a preferred embodiment, the enzyme is a lipase, such as Candida Antarctica lipase B, lipase IM (mucor meihei) lipase PS (pseudomonas cepacia), lipase PA (pseudomonas aeruginosa), lipase PF (pseudomonas fluorescence), lipase from Candida cylinderacea, porcina pancreatic lipase and lipase from aspergillus niger.

Furthermore, these enzymes selectively catalyze the esterification of carboxyl groups with less sterically hindered hydroxyl groups, such as the primary hydroxyl groups of the diol, under mild conditions, such as 50°C or lower. In other words, more sterically hindered hydroxyl groups, such secondary or tertiary hydroxyl groups that are also present in the polyol, are not or to a lower extent incorporated in the polycondensate due to their lower reactablity. Thus, the polycondensates show a linear or at least predominantly linear structure, which is particularly required for adhesive compositions. Furthermore, the polycondensate comprises at least one unreacted hydroxyl group per "polyol" unit in the polymer backbone. These functional groups in addition to the functional groups at the chain end may be used for further chemical reactions, such as a subsequent crosslinking of the polycondensate. Furthermore, the presence of these hydroxyl groups imparts a higher hydrophilicity, water solubility and biodegradability to the poylcondensate.

The absence of the enzyme in the condensation reaction between (a) and (b) does not lead to polycondensates showing a sufficiently high molecular weight and linear polymer structure and, thus, may not be applicable for the use in adhesive compositions due to their lower hydrophilicity, water solubility and biodegradability.

The catalyzing of the condensation reaction between (a) and (b) by an enzyme is particularly sufficient, when the compatibility of the dicarboxylic and the diol is good, i.e. when these compounds show a similar polarity. However, in a preferred embodiment, the condensation reaction of producing the carboxyl terminated oligomeric condensation product, i.e. the condensation reaction between (a1) and (a2), is also catalyzed by an enzyme. The enzyme may be preferable the same in both condensation reactions in order to minimize costs and purification steps. In such a case, both reaction steps may be performed in one vessel.

The polyol may be any compound that comprises at least three hydroxyl groups, wherein at least two of these hydroxyl groups are primary hydroxyl groups. The polyol comprises in embodiments from 3 to 20 carbon atoms, preferably from 4 to 10 carbon atoms, and/or the polyol comprises from 3 to 20 hydroxyl groups, preferably from 4 to 10 hydroxyl groups. In particular, the polyol may be a sugar alcohol, such as a compound selected from the group comprising sorbitol, erythritol, xylitol, glycerol, fructose, mannitol, or mixtures thereof. It is also preferred that the diol is selected from bio-based diols. The amount of the polyol may be from 5 to 40 wt%, more preferably from 10 to 20 wt%, based on the total amount of polycondensate. These embodiments ensure a sufficient amount of hydroxyl groups in the polycondensate, which in turn leads to an excellent ability for crosslinking, and which ensures an excellent hydrophilicity, water solubility and biodegradability.

The dicarboxylic acid may be any compound comprising at least two carboxylic groups. In embodiments, the dicarboxylic acid comprises a saturated dicarboxylic acid, comprising from 2 to 16 carbon atoms, preferably from 4 to 11 carbon atoms. The dicarboxylic acid may be selected from the group comprising fatty acid dicarboxylic acid, adipic acid, sebacic acid, suberic acid, succinic acid, pimellic acid, or mixtures thereof. It is also preferred that the dicarboxylic acid is selected from bio-based dicarboxylic acids.

In alternative embodiments, the dicarboxylic acid comprises a dimer of a fatty acid represented by the formula (i) HOOC-R₁-COOH, wherein R₁ is a linear or branched alkylene group that may contain one or more carbon double bonds, and wherein the dimer of the fatty acid preferably comprises 32 or more carbon atoms. A polycondensate comprising one or more monomers derived from a dimer of a fatty acid shows a highly flexible molecular structure, which in turn provides a low glass transition temperature (Tg) of preferably below 5°C, more preferable 0°C, most preferable -5°C. Such a low glass transition temperature of the polycondensate particularly enables the use for applications in adhesive compositions especially at such low temperatures.

The diol may be any compound comprising at least two, preferably two, primary hydroxyl groups. In embodiments, the diol comprises a saturated diol, comprising from 2 to 16 carbon atoms, preferably from 4 to 11 carbon atoms. The diol may be selected from the group comprising a dimer of a fatty alcohol, glycol, 1,4-butanediol or mixtures thereof. It is also preferred that the diol is selected from bio-based diols.

In alternative embodiments, the diol comprises a dimer of a fatty alcohol represented by the formula (ii) HO-R₂-OH, wherein R₂ is a linear or branched alkylene group that may contain one or more carbon double bonds and wherein the dimer of the fatty alcohol preferably comprises 32 or more carbon atoms.

The incorporation of at least a dimer of a fatty acid as the dicarboxylic acid and/or at least a dimer of a fatty alcohol as the diol ensures a sufficient flexibility of the polycondensate and, thus, a low transition temperature (Tg) of preferably below 5°C, more preferable 0°C, most preferable -5°C. Such a low glass transition temperature of the polycondensate particularly enables the use for applications in adhesive compositions especially at such low temperatures.

In particularly preferred embodiments, the polycondensate comprises monomers derived from dimers of fatty acids and/or dimers of fatty alcohols in combination with one or more further monomers derived from dicarboxylic acids and/or diols, wherein the monomers derived from dimers of fatty acids and the dimers of fatty alcohols show a higher amount of carbon atoms than the amount of carbon atoms of the dicarboxylic acids and diols. Preferably the difference in the amount of carbon atoms is 2 to 20 carbon atoms, more preferably from 6 to 12 carbon atoms. Polycarbonates with these mixtures of monomers show a sufficient balance of high molecular flexibility, low Tg, mechanic strength and adhesive properties.

In embodiments, the amount of dicarboxylic acid is from 20 to 95 wt%, preferably from 40 to 70 wt%, based on the total amount of polycondensate. The amount of diol may be from 20 to 95 wt%, preferably from 40 to 70 wt%, based on the total amount of polycondensate. In embodiments, when the dicarboxylic acid comprises a dimer of a fatty acid and/or when the diol comprises a dimer of a fatty alcohol, the amount of dicarboxylic acid and or diol is from 60 to 95 wt%, preferably from 80 to 90 wt%. These ranges provide polycondensates with a sufficient hydrophilicity, water solubility and biodegradability in combination with low production costs.

In addition to the monomers as identified above, the polycondensate may further comprise one or more repeating units, which are derived from a monomer selected from the group comprising lactones, hydroxyl acids, carbonates, amino alcohols, anhydrides, or mixtures thereof. In particular, the polycondensate may comprise monomers selected from the group comprising 12-hydroxystearic acid or mixtures thereof.

Any of the monomers as specified above may comprise further heteroatoms, such as O, S, N, or B, and/or may comprise unsaturated groups.

Furthermore, the weight average molecular weight of the oligomeric condensation product may range from 300 to 2000 g/mol, preferably 400 to 1500 g/mol. The weight average molecular weight of the polycondensate may range from 500 to 30000 g/mol, preferably 2000 to 20000 g/mol. A suitable polydispersity of the polycondensate may be from 1.2 to 3.0, preferably from 1.5 to 2.5. These ranges particular ensure the combination of favorable properties as identified above.

The method for producing a polycondensate in accordance with the present invention comprises the steps of: reacting (a1) a polyol comprising at least three hydroxyl groups, wherein at least two of these hydroxyl groups are primary hydroxyl groups, and (a2) a dicarboxylic acid, to obtain (a) a carboxyl-terminated an oligomeric condensation product, and subsequently reacting the oligomeric condensation product with (b) a diol, comprising two primary hydroxyl groups, to obtain the polycondensate, wherein the condensation reaction between (a) and (b) is catalyzed by an enzyme.

Preferably the method in accordance with the present invention is carried out in the absence of metal-based catalysts and/or metal containing additives such as accelerators, stabilizers etc., i.e. the reaction of (a) and (b) is carried out in the absence of such catalysts. More preferably also any other steps, such as the reaction of (a1) and (a2), are carried out in the absence of metal-based catalysts and/or metal containing additives such as accelerators, stabilizers etc..

The preferred embodiments and effects as specified above in connection with the polycondensate are also applicable for the method in accordance with the present invention.

In embodiments, the molar ratio of the carboxyl groups of the dicarboxylic acid and the primary hydroxyl groups of the polyol in the oligomerization reaction of (a2) with (a1) may be more than 1 : 1, preferably 2 : 1 or more, more preferably 3 : 1 or more. The adjustment of this molar ratio particularly facilitates the production of a carboxyl-terminated oligomer.

Furthermore, the molar ratio of the carboxyl groups of the carboxyl-terminated oligomeric condensation product and the primary hydroxyl groups of the diol in the condensation reaction of (a) and (b) may be more than 1 : 1, preferably 1.1 : 1 or more, more preferably 1.5 : 1 or more. This facilitates the production of linear polycondensates.

The pressure-sensitive adhesive composition is obtainable by reacting the polycondensate in accordance with the present invention with a crosslinking agent. The crosslinking agent is preferably selected from the group comprising isocyanates, epoxides, anhydrides, or mixtures thereof. In particular, the crosslinking agent is capable of reacting with hydroxyl groups and/or unsaturated groups of the polycondensate to produce a crosslinked polycondensate.

Moreover, the present invention is concerned with a hot melt adhesive composition comprising the polycondensate in accordance with the present invention. The hot melt adhesive composition preferably comprises a tackifier selected from the group comprising abietic acid, an abietic acid ester, a terpene resin, a terpene/phenol resin and a hydrocarbon resin and/or a wax selected from the group comprising a hydrogenated vegetable oil wax, in particular, hydrogenated castor oil.

The present invention and its favorable effects are illustrated by the following examples:

### Monomers

| | |
|---|---|
| S | D-Sorbitol (Sigma, CAS: 50-70-4, purity >98%) |
| SA | Sebacic Acid (Aldrich, CAS: 111-20-6, purity 99%) |
| FADA | fatty acid dimer diacid ("PRIPOL 1009" from CRODA; CAS: 68783-41-5) |
| BDO | 1,4-Butane Diol (Sigma, CAS: 110-63-4, purity 99%) |
| FADO | fatty acid dimer diol ("PRIPOL 2033" from CRODA; CAS: 147853-32-5) |
| CL | ε-caprolactone (Acros, CAS: 502-44-3, purity 99%) |

### Catalyst

Lipase "Novozym 435" supplied by Novozymes

### Measurement methods

1H and 13C NMR spectroscopy was performed on a Varian Mercury 400 MHz NMR in CDCl₃. Data were acquired using VNMR software. Chemical shifts are reported in ppm relative to tetramethylsilane (1H NMR).

Size exclusion chromatography (SEC) was performed on a Waters Alliance system equipped with a Waters 2695 separation module, a Waters 2414 refractive index detector (40 °C), a Waters 2487 dual absorbance detector and a Polymer Laboratories PLgel guard column followed by 2 PLgel 5mm Mixed-C columns in series at 40°C. Tetrahydrofuran (THF, Biosolve), stabilized with BHT, was used as eluent at a flow rate of 1 mL/min. The weight average molecular weight (Mw), the number average molecular weight (Mn) and the polydispersity index (PDI) were calculated against polystyrene standards (Polymer Laboratories, *Mₚ* = 580 Da up to *Mₚ* = 7.1 × 10⁶ Da). All samples were filtered through a 0.2 μm PTFE filter (13 mm, PP housing, Alltech) before analysis.

Differential Scanning Calorimetry (DSC) was performed on a TA Q100 DSC. 5 mg of dried polymer was weighed into aluminum hermetic pans. Temperature profiles from -80 °C up to 80 °C with a heating and cooling rate of 20 °C/min were applied. TA Universal Analysis software was used for data acquisition. Glass transition temperatures were determined from the second heating run.

Thermogravimetric analysis for evaluating the thermal stability was performed on a TA Q500. 5 mg of dried polymer was weighed into a ceramic pan. Temperature profiles from room temperature up to 600 °C with a heating rate of 10 °C/min were applied. TA Universal Analysis software was used for data acquisition.

The adhesive (or adhesion) strength was measured by a peel test at a peeling angle of 180° determining the force that is necessary to tear a strip of a tape from a PET surface at a constant speed. The result is the force referred to the width of the tape. Samples for the peel test were prepared as follows. At first, the adhesive composition was coated on a first PET carrier film and cured as described in examples 11 and 12. Then, 2 cm wide, 10 cm long piece of adhesive tapes were cut and placed on a table (the adhesive face on top). Half of the tape length was then covered with a second strip of a second PET plastic film, which is identical to the PET carrier film on which the adhesive composition has been previously coated. Then, this assembly was turn upside down and the free adhesive domain was applied manually on AB steel, PC PMMA or PP plates as a reference surface. A 2 kg cylinder was rolled twice on the tape in order to ensure a good contact between the adhesive and the reference surface. A dwell time of 15 minutes was observed prior to performing the peeling test. The peel test was performed on a Zwick Z005 testing machine with a constant peeling speed of 300 mm/min.

### EXAMPLES

### Example 1: Synthesis of the carboxyl-terminated oligomeric condensation product of sorbitol (S) and sebacic acid (SA) ("SA-S-SA")

Sebacic acid (76.87 g, 380 mmol) is added to a three-neck round-bottom flask and heated to 140 °C. Sorbitol (22.7 g, 125 mmol) is slowly added over 1 h to prevent coagulation. In total a 3:1 ratio of sabacic acid:sorbitol is obtained. When a homogeneous melt is obtained, the reaction is stirred for 6 h. The product is obtained as a 1:1 mixture of sebacic acid and SA-S-SA.

Results: Yield: 89%, Mn = 900 g/mol, PDI = 2.1. One possible structure of the SA-S-SA oligomer is shown below:

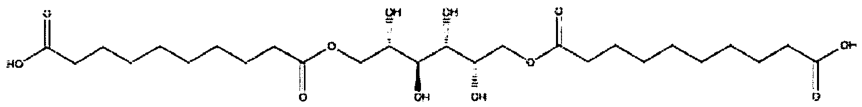

### Example 2: Synthesis of the carboxyl-terminated oligomeric condensation product of sorbitol (S) and the dimer of a fatty acid (FADA) ("FADA-S-FADA")

FADA (66.7 g, 110 mmol) is added to a three-neck round-bottom flask and heated to 140 °C. Sorbitol (6.6 g, 36.2 mmol) is slowly added over 1 h to prevent coagulation. In total a 3:1 ratio of FADA:sorbitol is obtained. When a homogeneous melt is obtained, the reaction is slowly cooled to 100 °C. 3 wt% of Novozym 435 is added and the reaction is stirred for 12 h. Afterwards the mixture is dissolved in THF and the enzyme is filtered off. After evaporation of the solvent the product is obtained. Results: Yield: 93% Mn = 1100 g/mol, PDI = 1.5. One possible structure of the FADA-S-FADA oligomer is shown below:

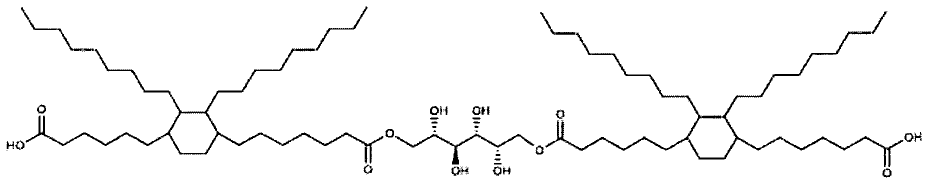

### Example 3: Synthesis of the carboxyl-terminated oligomeric condensation product of sorbitol (S) and adipic acid (AA) ("AA-S-AA")

Adipic acid (29.15 g, 160 mmol) is added to a three-neck round-bottom flask and heated to 150 °C. Sorbitol (70.64 g, 480 mmol) is slowly added over 1 h to prevent coagulation. In total a 3:1 ratio of adipic acid:sorbitol is obtained. When a homogeneous melt is obtained, the reaction is stirred for 4.5 h at 140 °C. The product is obtained as a 1:1 mixture of adipic acid and AA-S-AA. Results: Yield: 93%. One possible structure of the AA-S-AA oligomer is shown below:

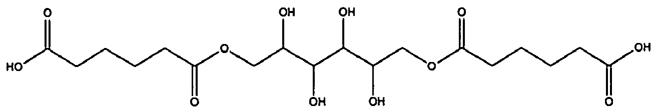

### Example 4: Polycondensation of the SA-S-SA oligomer with a dimer of a fatty diol (FADO)

The SA-S-SA oligomer has been previously prepared as described in example 1. The mixture of sebacic acid and SA-S-SA 14.62 g and FADO 10.38 g, 19 mmol are added to a three-necked round-bottom flask. The mixture is heated to 100 °C and 3 wt% Novozym 435 0.75 g, 3 wt% is added. The reaction is stirred for 48 hours. Afterwards the product is dissolved in the solvent THF and the enzyme is filtered off. After evaporation of the solvent the product is obtained. Results: Yield: 83%, Mn = 4000 g/mol, PDI = 1.9.

### Example 5: Polycondensation of the FADA-S-FADA oligomer with a dimer of a fatty diol (FADO)

The FADA-S-FADA oligomer has been previously prepared as described in example 2. The mixture of FADA and FADA-S-FADA obtained in the oligomerization step (19.54 g, 10.1 mmol) and FADO (5.46 g, 10.1 mmol) are added to a three-necked round-bottom flask. The mixture is heated to 100 °C and 3 wt% Novozym 435 0.75 g is added. The reaction is stirred for 24 hours under nitrogen flow. Afterwards the product is dissolved in THF and the enzyme is filtered off. After evaporation of THF the product is obtained. Results: Mn = 4700 g/mol, Mw = 7100 g/mol, PDI = 1.5; thermal stability: 10% wt loss: 338 °C.

### Example 6: Polycondensation of the FADA-S-FADA oligomer with a dimer of a fatty diol (FADO) and a caprolactone (CL)

The FADA-S-FADA oligomer has been previously prepared as described in example 2. The mixture of FADA and FADA-S-FADA obtained in the oligomerization step (18.59 g, 9.5 mmol), FADO (5.14 g, 9.5 mmol) and ε-caprolactone (1.09 g, 9.5 mmol) are added to a three-necked round-bottom flask. The mixture is heated to 100 °C and 3 wt% Novozym 435 0.75g is added. The reaction is stirred for 24 h under nitrogen flow. Afterwards the product is dissolved in THF and the enzyme is filtered off. After evaporation of THF the product is obtained. Results: Yield: 78%, Mn = 5900 g/mol, Mw=14200 g/mol, PDI = 2.4; thermal stability: 10% wt loss: 338°C. One possible structure of the polymeric product is shown below:

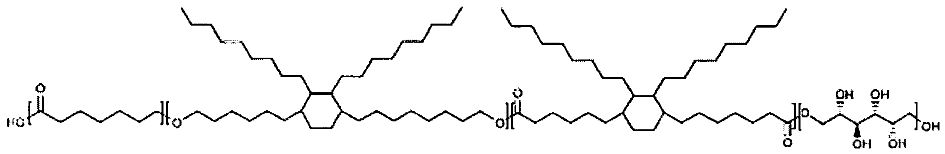

### Example 7: Polycondensation of the FADA-S-FADA oligomer with butanediol (BDO)

The FADA-S-FADA oligomer has been previously prepared as described in example 2. The mixture of FADA and FADA-S-FADA obtained in the oligomerization step (23.79 g, 12.3 mmol) and fatty acid dimer diol (1.11 g, 12.3 mmol) are added to a three-necked round-bottom flask. The mixture is heated to 100 °C and 3 wt% Novozym 435 0.75g is added. The reaction is stirred for 24 h under nitrogen flow. Afterwards the product is dissolved in THF and the enzyme is filtered off. After evaporation of THF the product is obtained. Results: Yield: 72%, Mn = 3500 g/mol, Mw = 5100 g/mol, PDI = 1.4; thermal stability: 10% wt loss: 322 °C. One possible structure of the polymeric product is shown below:

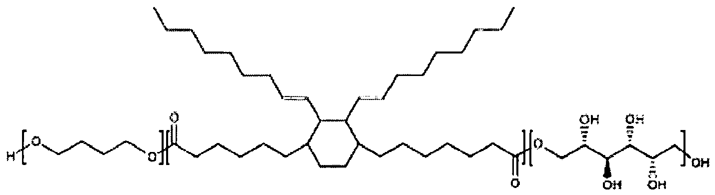

### Example 8: Polycondensation of the SA-S-SA oligomer with butanediol (BDO)

The SA-S-SA oligomer has been previously prepared as described in example 1. The mixture of sebacic acid and SA-S-SA obtained in the oligomerization step (14.62 g, 19 mmol) and BDO (10.38 g, 19 mmol) are added to a three-necked round-bottom flask. The mixture is heated to 100 °C and 3 wt% Novozym 435 0.75 g is added. The reaction is stirred for 24 h under nitrogen flow. Afterwards the products are dissolved in THF and the enzyme is filtered off. After evaporation of THF the product is obtained. Results: Yield: 75%, Mn = 3300 g/mol, Mw = 6700 g/mol, PDI = 2.1; thermal stability: 10% wt loss: 344 °C. One possible structure of the polymeric product is shown below:

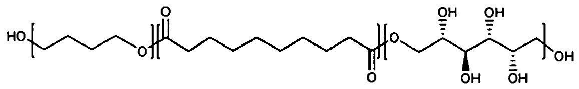

### Example 9: Polycondensation of the SA-S-SA oligomer with butanediol (BDO) and a dimer of a fatty diol (FADO)

The SA-S-SA oligomer has been previously prepared as described in example 1. The mixture of sebacic acid and SA-S-SA obtained in the oligomerization step (17.48 g, 23 mmol), FADO (6.20 g, 11.5 mmol) and 1,4-butanediol (1.03 g, 11.5 mmol) are added to a three-necked round-bottom flask. The mixture is heated to 100 °C and 3 wt% Novozym 435 0.75 g is added. The reaction is stirred for 24 h under nitrogen flow. Afterwards the product is dissolved in THF and the enzyme is filtered off. After evaporation of THF the product is obtained. Results: Yield: 71%, Mn = 3200 g/mol, Mw = 6600 g/mol, PDI = 2.1; thermal stability: 10% wt loss: 335 °C. One possible structure of the polymeric product is shown below:

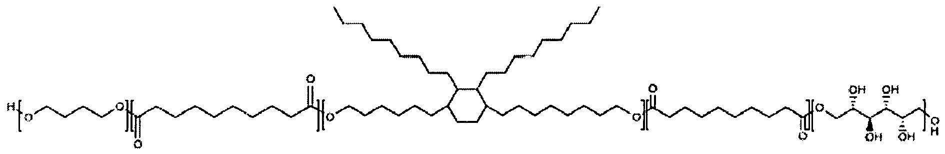

### Example 10: Polycondensation of the AA-S-AA oligomer with a dimer of a fatty alcohol (FADO)

The AA-S-AA oligomer has been previously prepared as described in example 3. The mixture of sebacic acid and AA-S-AA obtained in the oligomerization step (10.4 g, 17.8 mmol) and FADO (9.8 g, 17.8 mmol) are added to a three-necked round-bottom flask. The mixture is heated to 100 °C and 3 wt% Novozym 435 0.75 g is added. The reaction is stirred for 24 h under nitrogen flow. Afterwards the product is dissolved in THF and the enzyme is filtered off. After evaporation of THF the product is obtained. Results: Yield: 78%, Mn = 7800 g/mol, Mw = 17000 g/mol, PDI = 2.2; thermal stability: 10% wt loss: 328 °C.

### Example 11: Adhesive composition prepared from the enzyme-catalysed polyester and cured with an tri-functional isocyanate cross-linking agent

0.9992 g of the polycondensate synthesized in Example 6 are mixed with 0.0488 g of a tri-functional isocyanate compound Desmodur N3600 (from Bayer Material Siences). Once the solution is homogenised, an 80 μm adhesive layer is roll-coated manually at room temperature with a speed of 50 cm/min on a 50 μm thick PET film. This bilayer stack is cured in an oven at 130°C for 15 minutes.

This results in a tacky film suitable for pressure sensitive adhesive (PSA) applications. The thickness of the adhesive layer is 30 μm. The adhesive strength, as measured by a 180° peel test is 26.2 cN/20mm from BA Steel, 36.3 cN/20mm from polycarbonate (PC), 45.9 cN/20mm from poly(methyl methacrylate) (PMMA), and 20.9 cN/20mm from polypropylene (PP). The failure is interfacial on all substrates (i.e. no residue on the substrate after peel).

### Example 12: Adhesive composition prepared from the enzyme-catalysed polyester and cured with an tri-functional isocyanate cross-linking agent and a di-functional chain extender

0.51g of the polycondensate synthesized in Example 10 is mixed with 0.0022 g of a tri-functional isocyanate compound Desmodur N3600 (from Bayer Material Science), and 0.066 g of a dimer of a fatty isocyanate (trade name: DDI 1410). Once the solution is homogenised, a 30 μm adhesive layer is roll-coated manually at room temperature with a speed of 50 cm/min on a 50 μm thick PET film. This bilayer stack is cured in an oven at 130°C for 15 minutes.

This results in a tacky film suitable for pressure sensitive adhesive (PSA) applications. The thickness of the adhesive layer is 30 μm. The adhesive strength, as measured by a 180° peel test is 130 cN/20mm from BA Steel, 329.9 cN/20mm from polycarbonate (PC), 8.0 cN/20mm from poly(methyl methacrylate) (PMMA), and 114.3 cN/20mm from polypropylene (PP). The failure is interfacial on all substrates (i.e. no residue on the substrate after peel).

### Comparative Example 1: One-step polycondensation of sorbitol (S), a dimer of a fatty acid (FADA) and dimer of a fatty alcohol (FADO)

Sorbitol 0.28 g, 1.5 mmol, FADA 3.01 g, 5 mmol and FADO 1.95 g, 3.5 mmol are added to a three-necked round bottom flask in a ratio of primary hydroxyl groups to carboxyl groups of 1:1. The flask is heated to 120 °C to obtain a homogeneous melt. Afterwards the temperature is lowered to 100°C and 3 wt% Novozym 435 50 mg is added. The reaction mixture is put under argon flow and stirred for 24 h. Results: According to NMR analysis, a polycondensate of FADA and FADO is obtained, which does not contain any repeating units derived from sorbitol, Mn = 4000 g/mol, PDI = 1.6, Tg = -53.5 °C.

### Comparative Example 2: One-step polycondensation of sorbitol (S), sebacic acid (SA) and butanediol (BDO)

Sebacic acid (14.38 g, 35.6 mmol) and 1,4-butanediol (4.32 g, 24 mmol) were added to a three-necked round bottom flask at 120°C. Sorbitol (4.33 g, 11 mmol) was added slowly to the homogeneous mixture. The mixture was stirred until again a homogeneous mixture was obtained. The mixture was cooled to 100 °C and 3 wt% Novozym 435 (0.70 g) was added. The reaction mixture was put under argon flow and stirred for 24h. The mixture was cooled and THF was added to dissolve the product. The enzyme was filtered off and after evaporation of the solvent the product was obtained. Results: Yield: Mn = 11700 g/mol, Mw = 23000 g/mol, PDI = 2.0, Tg = no Tg observed, Tm = 38.4 °C, Tc = -14.9 °C

### Comparative Example 3: condensation reaction performed without an enzyme

The reaction of example 2 is repeated with the exception that no enzyme is added to the reaction mixture. IR data confirm that the carbonyl peak remains unchanged, which demonstrates that sorbitol and FADA do not undergo a condensation reaction without an enzyme.

### Conclusion:

Examples 1 to 10 clearly illustrate that the two-step method in accordance with the present invention provides polycondensates from a diol, a polyol and a dicarboxylic acid. In particular, this two-step method is suitable to produce polycondensates, wherein the diol and/or the dicarboxylic acid are derived from monomers of dimers of a fatty alcohol or a fatty acid, respectively. In contrast thereto, comparative examples 1 and 2 show that a known one-step method, i.e. a direct polycondensation, does not result in a polycondensation product comprising all monomers as repeating units. Comparative example 3 illustrates that the presence of an enzyme is necessary to achieve a condensation reaction between components (a) and (b). The polycondensates in accordance with the present invention can be used for pressure sensitive adhesive compositions such as demonstrated in examples 11 and 12.

## Claims

1. A polycondensate obtainable by reacting:
(a) a carboxyl-terminated oligomeric condensation product of (a1) a polyol comprising at least three hydroxyl groups, wherein at least two of these hydroxyl groups are primary hydroxyl groups, and (a2) a dicarboxylic acid, and
(b) a diol, comprising two primary hydroxyl groups,
wherein the condensation reaction between (a) and (b) is catalyzed by an enzyme.

2. The polycondensate according to claim 1, wherein the condensation reaction between (a1) and (a2) is catalyzed by an enzyme.

3. The polycondensate according to claims 1 or 2, wherein the polyol comprises from 3 to 20 carbon atoms, preferably from 4 to 10 carbon atoms, and/or wherein the polyol comprises from 3 to 20 hydroxyl groups, preferably from 4 to 10 hydroxyl groups.

4. The polycondensate according to claims 1 to 3, wherein the amount of the polyol is from 5 to 40 wt%, preferably from 10 to 20 wt%, based on the total amount of polycondensate.

5. The polycondensate according to claims 1 to 4, wherein the dicarboxylic acid comprises a saturated dicarboxylic acid, comprising from 2 to 16 carbon atoms, preferably from 4 to 11 carbon atoms, and/or wherein the diol comprises a saturated diol, comprising from 2 to 16 carbon atoms, preferably from 4 to 11 carbon atoms.

6. The polycondensate according to claims 1 to 5, wherein the dicarboxylic acid comprises a dimer of a fatty acid represented by the formula (i) HOOC-R₁-COOH, wherein R₁ is a linear or branched alkylene group that may contain one or more carbon double bonds, and wherein the dimer of the fatty acid preferably comprises 32 or more carbon atoms

7. The polycondensate according to claims 1 to 6, wherein the diol comprises a dimer of a fatty alcohol represented by the formula (ii) HO-R₂-OH, wherein R₂ is a linear or branched alkylene group that may contain one or more carbon double bonds and wherein the dimer of the fatty alcohol preferably comprises 32 or more carbon atoms.

8. The polycondensate according to claims 1 to 7, wherein the amount of dicarboxylic acid is from 20 to 95 wt%, preferably from 40 to 70 wt%, based on the total amount of polycondensate, and/or wherein the amount of diol is from 20 to 95 wt%, preferably from 40 to 70 wt%, based on the total amount of polycondensate.

9. The polycondensate according to claims 1 to 8, wherein the amount of dicarboxylic acid and or diol is from 60 to 95 wt%, preferably from 80 to 90 wt%, if the dicarboxylic acid comprises a dimer of a fatty acid and/or if the diol comprises a dimer of a fatty alcohol.

10. The polycondensate according to claims 1 to 9, further comprising one or more repeating units, which are derived from a monomer selected from the group comprising lactones, hydroxy acids, carbonates, amino alcohols, anhydrides, or mixtures thereof.

11. The polycondensate according to claims 1 to 10, wherein the weight average molecular weight of the oligomeric condensation product is 300 to 2000 g/mol, preferably 400 to 1500 g/mol and/or wherein the weight average molecular weight of the polycondensate is 500 to 30000 g/mol, preferably 2000 to 20000 g/mol and/or wherein the polydispersity of the polycondensate is 1.2 to 3.0, preferably 1.5 to 2.5.

12. A method of producing a polycondensate in accordance with claims 1 to 11, comprising the steps of: reacting (a1) a polyol comprising at least three hydroxyl groups, wherein at least two of these hydroxyl groups are primary hydroxyl groups, and (a2) a dicarboxylic acid, to obtain (a) a carboxyl-terminated an oligomeric condensation product, and subsequently reacting the oligomeric condensation product with (b) a diol, comprising two primary hydroxyl groups, to obtain the polycondensate, wherein the condensation reaction between (a) and (b) is catalyzed by an enzyme.

13. The method according to claim 11, wherein the molar ratio of the carboxyl groups of the dicarboxylic acid and the primary hydroxyl groups of the polyol in the oligomerization reaction of (a2) with (a1) is more than 1 : 1, preferably 2 : 1 or more, more preferably 3 : 1 or more, and/or wherein the molar ratio of the carboxyl groups of the carboxyl-terminated oligomeric condensation product and the primary hydroxyl groups of the diol in the condensation reaction of (a) and (b) is more than 1 : 1, preferably 1.1 : 1 or more, more preferably 1.5: 1 or more.

14. A pressure sensitive adhesive composition obtainable by reacting the polycondensate according to claims 1 to 11 with a crosslinking agent, which is preferably selected from the group comprising isocyanates, epoxides, anhydrides, or mixtures thereof.

15. A hot melt adhesive composition comprising the polycondensate according to claims 1 to 11, wherein the hot melt adhesive composition preferably comprises a tackifier, selected from the group comprising abietic acid, an abietic acid ester, a terpene resin, a terpene/phenol resin and a hydrocarbon resin and/or a wax selected from the group comprising a hydrogenated vegetable oil wax, in particular, hydrogenated castor oil.
